# EUROPÄISCHE PATENTSCHRIFT

(11) **EP 2 640 446 B1**
(45) Veröffentlichungstag und Bekanntmachung des Hinweises auf die Patenterteilung: **20.08.2014**
(21) Anmeldenummer: 12709023.1
(22) Anmeldetag: 10.02.2012
(51) Int. Cl.: A61M 5/24, A61M 5/315

(54) **KARPULENSPRITZE**
CARTRIDGE SYRINGE
SERINGUE À CARPULE

(30) Priorität: 23.02.2011 DE 102011012108
(43) Veröffentlichungstag der Anmeldung: 25.09.2013
(73) Patentinhaber: Camo Formen- Und Werkzeugbau GmbH, 4690 Schwanenstadt (AT)
(72) Erfinder: EIDLER, Josef, A-4690 Schwanenstadt (AT); CARNAL, André, CH-4500 Solothurn (CH)
(74) Vertreter: vP-IP von Puttkamer Berngruber Loth Spuhler
(86) Internationale Anmeldenummer: PCT/EP2012/052306
(87) Internationale Veröffentlichungsnummer: WO 2012/113661

(56) Entgegenhaltungen:
- EP-A1- 0 170 009
- WO-A1-02/04049
- WO-A1-03/101527
- GB-A- 2 445 228

## Beschreibung

Die Erfindung bezieht sich eine Karpulenspritze nach dem Oberbegriff des Anspruchs 1.

Eine solche Karpulenspritze ist aus GB 2 445 228 A bekannt. Dabei ist das vordere Ende des Stößels als korkenzieherförmige Spitze ausgebildet, welche durch Drehen des Stößels in den Stopfen der Karpule eingreift und damit den Stößel an dem Stopfen zur Aspiration fixiert. Das Einschieben des Stößels mit der korkenzieherförmigen Spitze ist jedoch umständlich, auch muss dafür Sorge getragen werden, dass sich der Stopfen in der Karpule beim Eindrehen der korkenzieherförmigen Spitze in den Stopfen nicht mit dreht.

In der Zahnmedizin werden fast ausschließlich Karpulenspritzen aus Metall verwendet, die nach der Applikation und vor dem nächsten Gebrauch neu sterilisiert, protokolliert und verblistert werden müssen. Dies ist mit einem erheblichen Aufwand verbunden. Zudem besteht die Gefahr, dass die Sterilisation nicht ordnungsgemäß durchgeführt wird und damit kontaminierte Spritzen zum Einsatz kommen.

Aufgabe der Erfindung ist es daher, eine Karpulenspritze bereitzustellen, die als einfach herstellbare Einweg-Spritze ausgebildet werden kann, wodurch sich eine Sterilisation nach dem Gebrauch erübrigt.

Dies wird erfindungsgemäß durch die im Anspruch 1 gekennzeichnete Karpulenspritze erreicht.

Das heißt, der Stößel besteht erfindungsgemäß aus einer Außenhülse, in der eine Seele angeordnet ist. Die Außenhülse und die Seele sind zwar gemeinsam verschiebbar, jedoch ist die Außenhülse gegenüber dem Aufnahmegehäuse drehbar, während die Seele drehfest mit dem hinteren Ende des Aufnahmegehäuses verbunden ist.

Am hinteren Ende der Außenhülse und damit des Stößels ist ein Betätigungselement, beispielsweise für den Daumen des Benutzers vorgesehen, z. B. ein Ring oder eine Platte.

Damit die Spritze zur Aspiration eingesetzt werden kann, also zum Absaugen von Körperflüssigkeit mit der Injektionskanüle, die an dem vorderen Ende des Aufnahmegehäuses befestigt ist, weist der Stopfen, mit dem der Ampullenzylinder der Karpule verschlossen ist, ein Sackloch auf, in das vordere Ende an der Außenhülse eingreift, das in dem Sackloch fixierbar ist.

Erfindungsgemäß weist das vordere Ende der Außenhülse dazu wenigstens einen nach innen, also zur Längsachse des Stößels federbelasteten Fixierhaken auf, der zwischen einer seitlich nach außen bewegten Fixierpositiön in dem Sackloch des Stopfens und einer nach innen bewegten Entriegelungsposition bewegbar ist.

Das Sackloch in dem Stopfen kann dabei eine glatte Bohrungswand aufweisen. Vorzugsweise ist sie jedoch mit wenigstens einer Ringnut oder Innengewinde versehen, in das der wenigstens eine Fixierhaken in der Fixierposition eingreift.

Um den Fixierhaken von der Fixierposition in die Entriegelungsposition zu bewegen und umgekehrt, ist am vorderen Ende der Seele ein Exzenter vorgesehen, der an dem Fixierhaken derart angreift, dass bei Drehung der Außenhülse der Fixierhaken entweder in die Fixierposition oder die Entriegelungsposition bewegbar ist. Der Fixierhaken ist also auf den Exzenter zu federbelastet.

Der Exzenter ist vorzugsweise im Querschnitt als Ellipse ausgebildet. Die in die Hauptachse der Ellipse gedrehten Fixierhaken nehmen die Fixierposition ein, während sie sich bei Drehung in die Nebenachse der Ellipse in der Entriegelungsposition befinden.

Das heißt, die beiden Fixierhaken werden zum Angriff an der Bohrüngswand bzw. zum Eingriff in die Ringnut oder das Innengewinde in dem Sackloch des Stopfens von dem im Querschnitt elliptischen Exzenter auseinander gespreizt, wenn die beiden Fixierhaken in die Hauptachse der Ellipse gedreht sind, während in der Freigabeposition die beiden Fixierhäken in der Nebenachse der Ellipse durch ihre Federbelastung aufeinander zu bewegt sind, sodass sie außer Eingriff mit der Bohrungswand des Sacklochs in dem Stopfen stehen, sodass nach Herausziehen des Ansatzes aus der Sackbohrung die Karpule nach ihrer Benutzung aus der seitlichen Längsöffnung in des Aufnahmegehäuses entnommen werden kann.

Zur Drehung der Außenhülse ist vorzugsweise eine Kulissenführung vorgesehen. Die Kulissenführung kann durch eine Aussparung in der Außenhülse gebildet sein, beispielsweise eine Nut an der Außenseite der Außenhülse oder einen schlitz in der Außenhülse, wobei in die Aussparung ein am hinteren Ende des Aufnahmegehäuses befestigter Vorsprung eingreift.

Die Aussparung kann beispielsweise aus zwei sich längs der Außenhülse erstreckenden Abschnitten bestehen, die durch einen schrägen Abschnitt miteinander verbunden sind.

Zur drehfesteh Führung der in der Außenhülse angeordneten Seele kann an der Seele eine sich in Längsrichtung erstreckende Rippe vorgesehen sein, die durch Vorsprünge an dem hinteren Ende des Aufnahmegehäuses beidseitig geführt ist. Stattdessen kann eine Längsaussparung, beispielsweise eine Längsnut in des Seele vorgesehen sein, in die ein am hinteren Ende des Aufnahmegehäuses befestigter Vorsprung eingreift.

Um die Spritze bei der Injektion mit dem Zeige- und Mittelfinger zu halten, weist das Aufnahmegehäuse vorzugsweise am hinteren Ende eine plattenförmige Verbreiterung auf. Am vorderen Ende des Aufnahmegehäuses ist vorzugsweise ein Gewinde vorgesehen, um die in das Aufnahmegehäuse ragende Durchstechkanüle zum Durchstechen der Durchstechmembran der Karpule und die Injektionskanüle zu befestigen, wobei beide Kanülen vorzugsweise eine Einheit bilden.

Damit die erfindungsgemäße Karpulenspritze als Einwegspritze einsetzbar ist, besteht sie vorzugweise aus Kunststoff, wobei sie vorzugsweise nach einem Mehrkomponenten-Montage-Spritzgießverfahren hergestellt wird.

Dabei kann ein Spritzwerkzeug mit mehreren Spritzeinheiten, z. B. vier Spritzeinheiten, verwendet werden, die jeweils mehrere Formnester besitzen.

Das Spritzwerkzeug wird vorzugsweise durch Drehen über wenigstens drei Stationen bewegt, wobei in der ersten Station das Aufnahmegehäuse und das Betätigungselement gespritzt werden und in der zweiten Station die Außenhülse, die das Aufnahmegehäuse mit dem Betätigungselement verbindet, während in der dritten Station die Seele in die Außenhülse gespritzt wird, und zwar aus einem Kunststoff, der an dem Kunststoff der Außenhülse nicht haftet, also insbesondere mit dem Kunststoff der Außenhülse nicht reagiert.

vorzugsweise ist eine vierte Station vorgesehen, an der die fertigen Spritzen entnommen werden, worauf sie z. B. mit einer verpackungsmaschine in eine Folie verpackt werden.

Das Spritzen in den einzelnen Stationen bzw. die Entnahme der Spritzen an der vierten Station erfolgt gleichzeitig. Vorzugsweise wird das Spritzwerkzeug durch Drehen von einer zur anderen Station bewegt.

Nachstehend ist die Erfindung anhand der beigefügten Zeichnung beispielhaft näher erläutert. Darin zeigen:
Figur 1 eine perspektivische Darstellung der Karpulenspritze;
Figur 2 und 3 jeweils die hinteren Teile des Aufnahmegehäuses mit dem vorderen Teil der Stößels mit den federbelasteten Fixierhaken an der Außenhülse in der Entriegelungsposition bzw. der auseinander gespreizten Fixierposition;
Figur 4 einen Längsschnitt der Karpulenspritze;
Figur 5 einen Schnitt entlang der Linie V-V in Figur 4;
Figur 6 den Ausschnitt C in Figur 4 in vergrößerter Wiedergabe;
Figur 7 eine Seitenansicht der Karpulenspritze;
Figur 8 und 9 jeweils einen Längsschnitt durch die Karpulenspritze mit den Fixierhäken in der Entriegelungsposition bzw. Fixierposition;
Figur 10 und 11 eine vergrößerte Darstellung des Bereiches A bzw. B in Figur 8 und 9; und

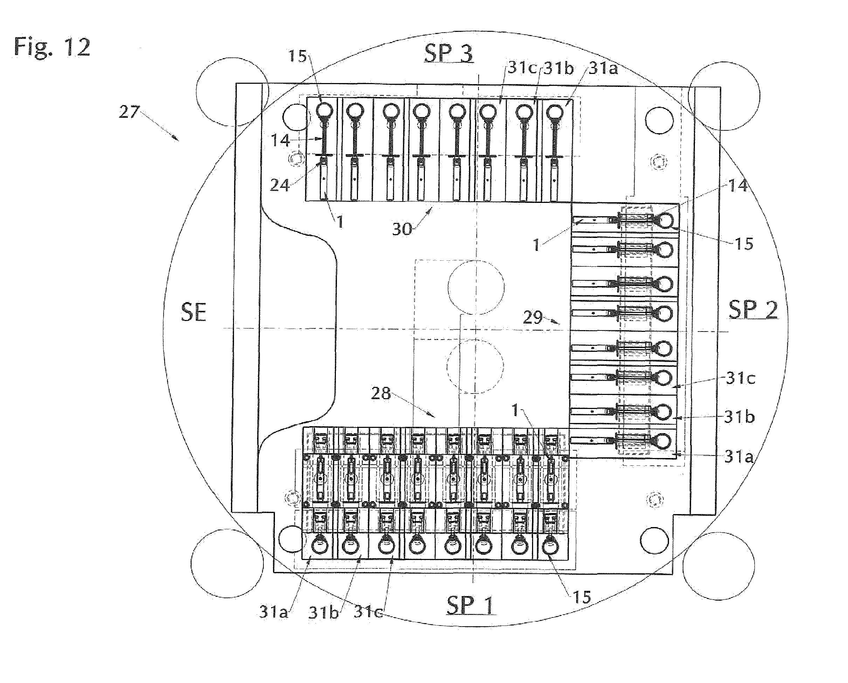

Figur 12 eine Draufsicht auf das Spritzwerkzeug zur Herstellung der Karpulenspritze.

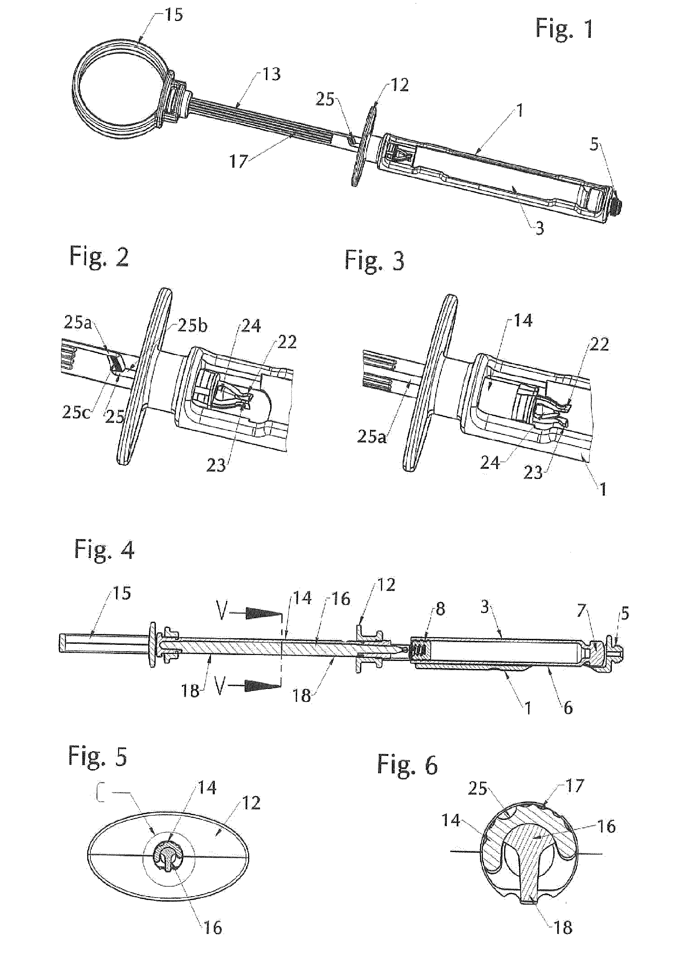

Gemäß Figur 1, 4, 7, 8 und 9 weist die Karpulenspritze ein zylindrisches Aufnahmegehäuse 1 auf, das mit einer seitlichen Längsöffnung 2 versehen ist, um die Karpule 3 in das Aufnahmegehäuse 1 zu legen.

Am vorderen Ende des Aufnahmegehäuses 1 ist eine Axialbohrung 4 mit einem Gewinde 5 vorgesehen, um eine nicht dargestellte Einheit aus Durchstechkanüle, die durch die Bohrung 4 in das Aufnahmegehäuse 1 ragt, und Injektionskanüle aufzuschrauben.

Die Karpule 3 wird durch einen mit einem Medikament gefüllten Ampullenzylinder 6, eine Durchstechmembran 7 am vorderen Ende und einen Stopfen 8 am hinteren Ende gebildet.

Die Durchstechmembran 7 wird beim Einlegen der Karpule 3 in das Aufnahmegehäuse 1 durch die an dem Gewinde 5 befestigte, nicht dargestellte Durchstechkanüle durchstochen.

Der Stopfen 8 weist ein Sackloch 9 auf, das mit einem Innengewinde 11 oder dergleichen Nuten am Umfang versehen ist.

Am hinteren Ende des Aufnahmegehäuses 2, das mit einer plattenförmigen Verbreiterung 12 versehen ist, ist ein Stößel 13 verschiebbar geführt. Mit dem Stößel 13 wird das Medikament zur Injektion über die nicht dargestellte Durchstechkanüle und die daran befestigte, nicht dargestellte Injektionskanüle mit dem Stopfen 8 aus dem Ampullenzylinder 6 gepresst.

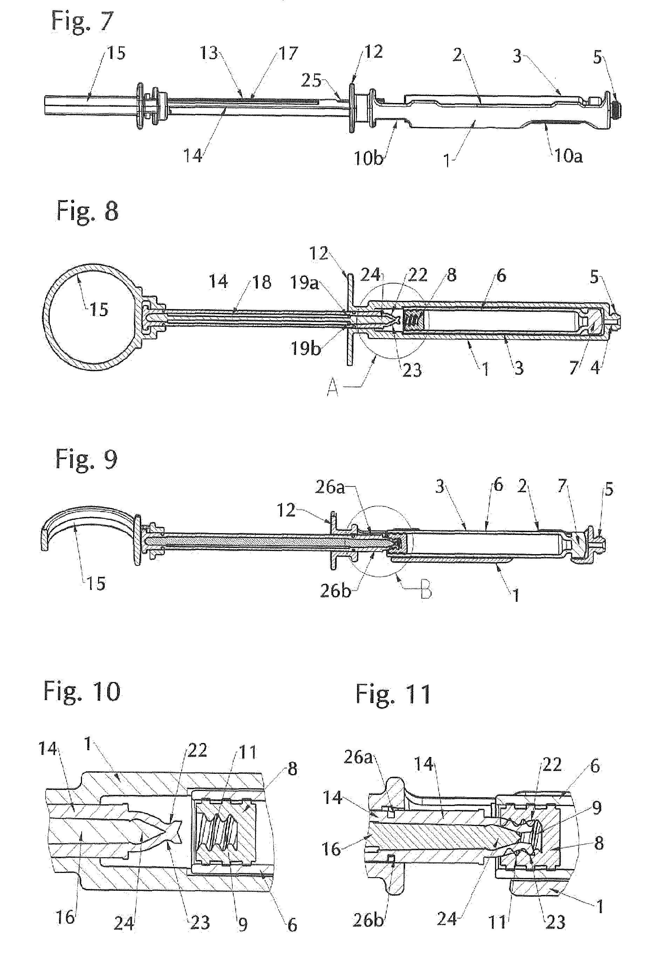

Der Stößel 13 besteht aus einer Außenhülse 14, an der das z. B. als Ring ausgebildete Betätigungselement 15 befestigt ist, sowie eine Seele 16, die in der Außenhülse 14 angeordnet ist. Die Außenhülse 14 ist um ihre Längsachse gegenüber dem Aufnahmegehäuse 1 drehbar. Sie ist dazu mit einer rauen Oberflächenstruktur, beilspielsweise einer Riffelung 17 versehen. An der der Langsöffnung 2 gegenüberliegenden Seite weist das Aufnahmegehäuse 1 zwei Fenster 10a, 10b am vorderen bzw. hinteren Endbereich auf (Figur 7).

Die Seele 16 ist zwar zusammen mit der Außenhülse 14 verschiebbar an dem hinteren Ende des Aufnahmegehäuses 1 geführt, jedoch drehfest angeordnet, also im Gegensatz zur Außenhülse 14 gegenüber dem Aufnahmegehäuse 1 nicht drehbar.

Dazu weist die Seele 16 eine sich längs erstreckende Rippe 18 auf, die durch stiftförmige Vorsprünge 19a, 19b, die am hinteren Ende des Aufnahmegehäuses 1 in Höhe der Verbreiterung 12 befestigt sind, beidseitig geführt wird (Figur 8).

Zur Aspiration weist das vordere Ende der Außenhülse 14 als Ansatz zwei nach innen, also aufeinander zu federbelastete Fixierhaken 22, 23 auf, die zwischen einer seitlich nach außen bewegten, also auseinander gespreizten Fixierposition (Figur 3 und 11) und einer nach innen bewegten Entriegelungsposition (Figur 2 und 10) bewegbar sind.

Zur Bewegung der Fixierhaken 22, 23 in die Fixierposition und die Entriegelungsposition und umgekehrt ist ein Exzenter 24 am vorderen Ende der Seele 16 vorgesehen.

Der sich nach vorne verjüngende Exzenter 24 ist im Querschnitt als Ellipse ausgebildet. Bei durch Drehen der Außenhülse 14 in die Hauptachse der Ellipse gedrehten Fixierhaken 22, 23 sind die Fixierhaken 22, 23 so auseinander gespreizt, dass sie in die Gewindenut des Innengewindes 11 eingreifen (Figur 9 und 11), während dann, wenn die Außenhülse 14 gegenüber der Seele 16 so gedreht ist, dass die Fixierhaken 22, 23 in der Nebenachse der Ellipse an dem Exzenter 24 anliegen, die Fixierhaken 22, 23 außer Eingriff mit der Gewindenut des Innengewindes 11 bringbar sind (Figur 8 und 10).

Um die Außenhülse 14 gegenüber der Seele 16 um 90°, also so zu drehen, dass die Fixierhaken 22, 23 an dem Exzenter 24 von der Hauptachse zu der Nebenachse der Ellipse bewegt werden und umgekehrt, ist eine Kulissenführung vorgesehen.

Die Kulissenführung besteht, wie z. B. in Figur 2 dargestellt, aus jeweils einer Nut 25 an der Außenseite auf beiden Seiten der Außenhülse 14. Wie anhand der in Figur 2 dargestellten einen Nut 25 ersichtlich, besteht die Nut 25 aus zwei parallel in Längsrichtung verlaufenen Abschnitten 25a, 25b, die durch einen schrägen Abschnitt 25c verbunden sind.

In die bzw. jede Nut 25 greift ein stiftförmiger Vorsprung 26a, 26b ein, der an dem Aufnahmegehäuse 1 befestigt ist (Figur 9 und 11).

Die erfindungsgemäße Karpulenspritze wird durch Spritzgießen aus Kunststoff hergestellt.

Dabei wird ein Mehrkomponenten-Montage-Spritzgießverfahren angewendet, für das das in Figur 9 verwendete Spritzgußwerkzeug 37 benutzt wird.

Das Spritzgußwerkzeug 27, das um eine zur Zeichenebene senkrechte Achse drehbar ist, besteht aus vier Spritzeinheiten, die jeweils mit mehreren, beispielsweise acht Formnestern 31a, 31b, 31c... versehen sind. Dabei sind in der Zeichnung nur die drei Spritzeinheiten 28, 29, 30 zu sehen, während die vierte Spritzeinheit hinter der Spritzeinheit 28 angeordnet ist.

In der Spritzstation S1 werden in der Spritzeinheit 28 und der nicht dargestellten dahinter liegenden Spritzeinheit das Aufnahmegehäuse 1 mit Gewinde 5, der Verbreiterung 18 und den Vorsprüngen 19a, 19b, 26a, 26b sowie das ringförmige Betätigungselement 15 gespritzt.

In der zweiten Station S2 werden in der Spritzeinheit 29 die mit den federbelasteten Fixierhaken 22, 23 versehene Außenhülse 14 gespritzt, die das Aufnahmegehäuse 1 mit dem Betätigungselement 15 verbindet. Dabei wird die Außenhülse 14 aus einem Kunststoff gespritzt, der an dem Kunststoff, aus dem das Aufnahmegehäuse 1 und das Befestigungselement 15 besteht, nicht haftet.

In der dritten Station S3 wird in der Spritzeinheit 30 die Seele 16 mit Rippe 18 und mit dem Exzenter 24 am vorderen Ende gespritzt, und zwar aus einem Kunststoff, der an dem Kunststoff, aus dem die Außenhülse 14 besteht, nicht haftet.

An der Station SE werden die fertigen Karpulenspritzen entnommen und dann einer nicht dargestellten Verpackungseinheit zur sterilen Verpackung zugeführt.

Das Spritzwerkzeug 27 wird also nach jedem Spritzvorgang um 90° gedreht.

Auch ist ersichtlich, das erfindungsgemäß das Mehrkomponenten-Montage-Spritzen gegebenenfalls mit nur zwei Komponenten oder Kunststoffen durchgeführt werden kann, nämlich mit einer Komponente für die Seele 16, das Aufnahmegehäuse 1 und das Betätigungselement 15 und einer weiteren Komponente für die Außenhülse 14.

## Patentansprüche

1. Karpulenspritze mit einem Aufnahmegehäuse (1) mit einer Längsöffnung (2) zum Einlegen der Karpule (3), welche einen mit einem Medikament gefüllten Ampullenzylinder (6) aufweist, der am vorderen Ende mit einer Durchstechmembran (7) und an hinteren Ende mit einem Stopfen (8) verschlossen ist, und mit einem am hinteren Ende des Aufnahmegehäuses (1) verschiebbar geführten Stößel (13), der nach Durchstechen der Durchstechmembran (7) den Stopfen (8) zur Infektion des Medikaments in den Ampullenzylinder (6) presst, wobei des hintere Ende des Stößels (13) mit einem Betätigungselement (15) versehen ist und das vordere Ende des Stößels (13) zur Aspiration an dem Stopfen (8) fixierbar ist, wobei Stößel (13) eine Außenhülse (14) aufweist, in der eine Seele (16) angeordnet ist, **dadurch gekennzeichnet, dass** die Außenhülse (14) drehbar und die Seele (16) drehfest am hinteren Ende des Aufnahmegehäuses (1) verschiebbar geführt sind, des vordere Ende der Außenhülse (14) zur Fixierung in einem Sackloch (9) des Stopfens (8) wenigstens einen nach innen federbelasteten Fixierhaken (22, 23) aufweist, der zwischen der seitlich nach außen bewegten Fixierposition in dem Sackloch (9) des Stopfens (8) und einer nach innen bewegten Entriegelungsposition bewegbar ist, und an dem vorderen Ende der Seele (16) eine Exzenter (24) vorgesehen ist, der an dem Fixierhaken (22, 23) derart angreift, dass bei Drehung der Außenhülse (14) der Fixierhaken (22, 23) entweder in die Fixierposition oder die Entriegelungsposition bewegbar ist.

2. Karpulenspritze nach Anspruch 1, **dadurch gekennzeichnet, dass** zwei gegenüberliegende federbelastete Fixierhaken (22, 23) vorgesehen sind und der Exzenter (24) im Querschnitt als Ellipse ausgebildet ist, wobei die in der Hauptachse der Ellipse gedrehten Fixierhaken (22, 23) in die Fixierposition und die in die Nebenachse der Ellipse gedrehten Fixierhaken (22, 23) in die Entriegelungsposition gedreht sind.

3. Karpulenspritze nach Anspruch 1 oder 2, **dadurch gekennzeichnet, dass** zur Drehung der Außenhülse (14) eine Kulissenführung vorgesehen ist.

4. Karpulenspritze nach Anspruch 3, **dadurch gekennzeichnet, dass** die Kulissenführung durch eine Aussparung (25) an der Außenseite der Außenhülse (14) gebildet wird, in die ein am hinteren Ende des Aufnahmegehäuses 81) befestigter Vorsprung (26a, 26b) eingreift.

5. Karpulenspritze nach Anspruch 1, **dadurch gekennzeichnet, dass** die Seele (16) zur drehfesten Führung eine sich längs erstreckende Rippe (18) aufweist, die durch Vorsprünge (19a, 19b) am hinteren Ende des Aufnahmegehäuses (1) beidseitig geführt ist.

6. Karpulenspritze nach einem der vorherigen Ansprüche, **dadurch gekennzeichnet, dass** sie aus Kunststoff besteht und als Einwegspritze ausgebildet ist.

7. Verfahren zur Herstellung der Karpulenspritze nach Anspruch 6, **dadurch gekennzeichnet, dass** ein Mehrkomponenten-Montage-Spritzgießverfahren angewendet wird.

8. Verfahren nach Anspruch 7, **dadurch gekennzeichnet, dass** in einer ersten Station (S1) das Aufnahmegehäuse (1) und das Betätigungselement (15), in einer zweiten Station (S2) die Außenhülse (14), die das Aufnahmegehäuse (1) mit dem Betätigungselement (15) verbindet, wobei die Außenhülse (14) aus einem Kunststoff gespritzt wird, der an dem Kunststoff des Aufnahmegehäuses (1) und des Befestigungselements (15) nicht haftet, und in einer dritten Station (S3) die Seele (16) in die Außenhülse (14) aus einem an dem Kunststoff der Außenhülse (14) nicht haftenden Kunststoff gespritzt wird.

## Claims

1. Cartridge syringe having a receptacle housing (1) with a longitudinal opening (2) for inserting the cartridge (3) having an ampule cylinder (6) filled with a medication and closed at the front end by means of a piercing membrane (7) and at the rear end by means of a stopper (8), and with a displaceably guided ram (13) at the rear end of the receptacle housing (1), pressing the stopper (8) for injecting the medication in the ampule cylinder (6) after piercing the piercing membrane (7), wherein the rear end of the ram (13) is provided with an actuating element (15) and the front end of the ram (13) can be attached to the stopper (8) for aspiration, wherein the ram (13) has an outer sleeve (14) in which a core (16) is disposed, **characterised in that**, at the rear end of the receptacle housing (1), the outer sleeve (14) is displaceably guided in a rotatable manner and the core (16) is displaceably guided in a rotationally fixed manner, that the front end of the outer sleeve (14) has, for the attachment in a bind hole (9) of the stopper (8), at least one fixing hook (22, 23) which is spring-loaded inwardly and which is movable between the fixing position being directed outwardly sideways in the blind hole (9) of the stopper (8) and an inwardly directed unlocked position, and that, at the front end of the core (16), a cam (24) is provided, engaging with the fixing hook (22, 23) such that, upon rotation of the outer sleeve (14), the fixing hook (22, 23) is movable either to the fixing position or to the unlocked position.

2. Cartridge syringe according to claim 1, **characterised in that** two spring-loaded fixing hooks (22, 23) facing each other are provided and the cam (24) is elliptical in cross section, wherein the fixing hooks (22, 23) rotated in the major axis of the ellipse are rotated to the fixing position and the fixing hooks (22, 23) rotated in the minor axis of the ellipse are rotated to the unlocked position.

3. Cartridge syringe according to claim 1 or 2, **characterised in that** a slotted guide is provided for the rotation of the outer sleeve (14).

4. Cartridge syringe according to claim 3, **characterised in that** the slotted guide is formed by means of a recess (25) on the outside of the outer sleeve (14), with a protrusion (26a, 26b) attached to the rear end of the receptacle housing (1) engaging therewith.

5. Cartridge syringe according to claim 1, **characterised in that**, in order to be guided In a rotationally fixed manner, the core (16) has a longitudinally extending rib (18) which is guided on both sides by means of protrusions (19a, 19b) at the rear end of the receptacle housing (1).

6. Cartridge syringe according to one of the preceding claims, **characterised in that** it is made of a plastic material and configured as a disposable syringe.

7. Process for producing the cartridge syringe according to claim 6, **characterised In that** a multi-component injection moulding process Is applied.

8. Process according to claim 7, **characterised in that** the receptacle housing (1) and the actuating element (15) are injection-moulded at a first station (51), the outer sleeve (14) connecting the receptacle housing (1) to the actuating element (15) is injection moulded at a second station (S2), wherein the outer sleeve (14) is injection-moulded from a plastic material which does not adhere to the plastic material of the receptacle housing (1) and the fastening element (15), and, at a third station (S3), the core (16) is injection-moulded in the outer sleeve (14) from a plastic material which does not adhere to the plastic material of the outer sleeve (14).

## Revendications

1. Seringue à carpule comportant un boîtier de réception (1) doté d'une ouverture longitudinale (1) pour introduire la carpule (3) qui présente un cylindre d'ampoule (6) rempli d'un médicament et fermé à l'extrémité avant par une membrane à perforer (7) et à l'extrémité arrière par un bouchon (8), et comportant un poussoir (13) guidé de manière coulissante à l'extrémité arrière du boîtier de réception (1), qui, après avoir perforé la membrane à perforer (7), presse le bouchon (8) pour injecter le médicament dans le cylindre d'ampoule, l'extrémité arrière du poussoir (13) étant pourvue d'un élément d'actionnement (15) et l'extrémité avant du poussoir (13) pouvant être fixée pour aspirer au niveau du bouchon (8), le poussoir présentant une gaine extérieure (14) dans laquelle est agencée une âme (16), **caractérisée en ce que** la gaine extérieure (14) est guidée de manière coulissante en rotation et l'âme (16) est guidée de manière coulissante solidaire en rotation à l'extrémité arrière du boîtier de réception (1), **en ce que** l'extrémité avant de la gaine extérieure (14) présente, pour être fixée dans un trou borgne (9) du bouchon (B), au moins un crochet de fixation (22, 23) sollicité par ressort vers l'intérieur, qui peut se déplacer entre la position de fixation mue latéralement vers l'extérieur dans le trou borgne (9) du bouchon (8) et une position de déverrouillage mue vers l'intérieur, et **en ce qu'**à l'extrémité avant de l'âme (16) est prévu un excentrique (24) qui attaque le crochet de fixation (22, 23) de telle sorte que lors de la rotation de la gaine extérieure (14), le crochet de fixation (22, 23) peut être déplacé soit dans la position de fixation soit dans la position de déverrouillage.

2. Seringue à carpule selon la revendication 1, **caractérisée en ce que caractérisée en ce qu'**il est prévu deux crochets de fixation (22, 23) opposés l'un à l'autre et sollicités par ressorts et **en ce que** l'excentrique (24) est réalisé sous forme d'ellipse en section transversale, les crochets de fixation (22, 23) tournés dans le grand axe de l'ellipse étant tournés jusque dans la position de fixation et les axes de fixation (22, 23) tournés dans le petit axe de l'ellipse étant tournés jusque dans la position de déverrouillage.

3. Seringue à carpule selon la revendication 1 ou 2, **caractérisée en ce qu'**un guidage à coulisse est prévu pour la rotation de la gaine extérieure (14).

4. Seringue à carpule selon la revendication 3, **caractérisée en ce que** le guindage à coulisse (14) est formé par un évidement (25) sur la face extérieure de la gaine extérieure (14), dans lequel s'engage une saillie (26a, 26b) fixée à l'extrémité extérieure du boîtier de réception (1).

5. Seringue à carpule selon la revendication 1, **caractérisée en ce que** l'âme (16) pour le guidage solidaire en rotation présente une nervure (18) à extension longitudinale qui est guidée des deux côtés à l'extrémité postérieure par des saillies (19a, 19b).

6. Seringue à carpule selon l'une des revendications précédentes, **caractérisée en ce qu'**elle est en matière plastique et est réalisée sous forme de seringue à usage unique.

7. Procédé de fabrication d'une seringue à carpule selon la revendication 6, **caractérisé en ce qu'**il est mis en oeuvre un procédé de moulage par injection de multicomposants.

8. Procédé selon la revendication 7, **caractérisé en ce que** dans une première station (S1) sont moulés le boîtier de réception (1) et l'élément d'actionnement (15), dans une deuxième station (S2) est moulée la gaine extérieure (14) qui relie le boîtier de réception (1) à l'élément d'actionnement (15), la gaine extérieure (14) étant moulée dans une matière plastique qui n'adhère pas à la matière plastique du boîtier de réception (1) et de l'élément d'actionnement (15), et dans une troisième station (S3), l'âme (16) est moulée dans la gaine extérieure (14) dans une matière plastique qui n'adhère pas à la matière plastique de la gaine extérieure (14).
